# EUROPEAN PATENT SPECIFICATION

(11) **EP 1 407 272 B1**
(45) Date of publication and mention of the grant of the patent: **12.09.2007**
(21) Application number: 02752236.6
(22) Date of filing: 10.07.2002
(51) Int. Cl.: G01N 33/58, G01N 33/92, G01N 33/533, G01N 33/542

(54) **FLUORESCENCE POLARIZATION-BASED HOMOGENOUS ASSAY FOR AFLATOXINS**
AUF FLUORESZENZPOLARISATION BERUHENDER HOMOGENER ASSAY FÜR AFLATOXINE
DOSAGE HOMOGENE BASE SUR LA POLARISATION DE FLUORESCENCE UTILISE POUR DETECTER LES AFLATOXINES

(30) Priority: 13.07.2001 US 905452
(43) Date of publication of application: 14.04.2004
(73) Proprietor: Diachemix LLC, Milwaukee, WI 53202 (US)
(72) Inventor: NASIR, Mohammad, Sarwar, Grayslake, IL 60030 (US); JOLLEY, Michael, E., Round Lake, IL 60073 (US)
(74) Representative: Schnappauf, Georg
(86) International application number: PCT/US2002/021696
(87) International publication number: WO 2003/006994

(56) References cited:
- WO-A1-97/19950
- US-A- 4 444 879
- US-A- 5 262 333
- US-A- 5 380 825
- US-A- 5 427 960
- HOLLADAY S D ET AL: "EVALUATION OF AN INDIRECT ELISA FOR SCREENING ANTIBODY AGAINST AFLATOXINS" AMERICAN JOURNAL OF VETERINARY RESEARCH, vol. 52, no. 2, 1991, pages 222-223, XP008049680 ISSN: 0002-9645
- MOHAMMED SARWAR NASIR ET AL: "Fluorescence Polarization: an analytical tool for immunoassay and drug discovery" COMBINATORIAL CHEMISTRY AND HIGH THROUGHPUT SCREENING, HILVERSUM, NL, vol. 2, no. 4, 1999, pages 177-190, XP001062258 ISSN: 1386-2073
- MARAGOS C M ET AL: "FLUORESCENCE POLARIZATION AS A MEANS FOR DETERMINATION OF FUMONISINS IN MAIZE" JOURNAL OF AGRICULTURAL AND FOOD CHEMISTRY, AMERICAN CHEMICAL SOCIETY. WASHINGTON, US, vol. 49, no. 2, February 2001 (2001-02), pages 596-602, XP001058007 ISSN: 0021-8561
- NASIR MOHAMMAD SARWAR ET AL: "Development of a fluorescence polarization assay for the determination of aflatoxins in grains" JOURNAL OF AGRICULTURAL AND FOOD CHEMISTRY, vol. 50, no. 11, 22 May 2002 (2002-05-22), pages 3116-3121, XP002335467 ISSN: 0021-8561
- DATABASE BIOSIS [Online] HOLLADAY S.D.: 'Preparation of protein conjugatable aflatoxin oxime derivative from significantly reduced starting quantities', XP002956856 Retrieved from STN Database accession no. 1990:77996 & VETERINARY HUMAN TOXICOLOGY vol. 31, no. 6, 1989, pages 526 - 527, 624
- DATABASE CANCERLIT [Online] HOLLADAY S.D.: 'Production of specific antibody against aflatoxin M1', XP002956857 Retrieved from STN Database accession no. 90665835 & DISSERTATIONS ABSTRACTS INTERNATIONAL vol. 50, no. 10, 1990, page 4298
- CAMPBELL AND STOLOFF: "Implications of mycotoxins for human health." J. AGR. FOOD CHEM,, vol. 22, 1974, pages 1006-1015,

## Description

### BACKGROUND OF THE INVENTION

### 1. Field of the Invention

This invention relates to the field of mycotoxin assays. More particularly, this invention relates to a homogeneous assay that uses changes in fluorescence polarization to detect the presence of aflatoxins in agricultural products.

### 2. Description of Related Art

Aflatoxins are mycotoxins produced by *Aspergillus flavus* molds¹. Aflatoxins have been known for a long time, but their carcinogenicity was first detected in the late 1960s⁴. Various forms of aflatoxin, including B₁, B₂, G₁, and G₂ and many others, have been found in many forms of human foods, such as cereals, grains and peanut products^{9,11}. Aflatoxin B₁ is the most toxic and most abundant of all. An exposure to aflatoxins has been associated with an increased incidence of primary hepatocellular carcinoma⁷.

Due to their toxicity and carcinogenicity, various analytical methods have been devised to quantitatively determine the amount of aflatoxin in agricultural products^{1-4,6,8}. One difficulty with such assays is that aflatoxins are very hydrophobic and therefore very insoluble in aqueous solvents. Thus, mixtures of organic solvent with water have generally been used to extract aflatoxins from samples.

Another difficulty is that most of the common assays, including TLC and HPLC¹⁰, require extended cleanup steps and derivatization after extraction in order to get rid of interfering substances. ELISA methods are relatively faster but are hard to quantify due to various washing steps, liquid transfers and incubation times and cleaning steps (e.g. Holladay et. al, Amer. J. V et. Res., V52, p222-223).

Accordingly, there is a need for an assay for the determination of aflatoxins that is rapid, simple to apply, and that can yield quantitative results.

### SUMMARY OF THE INVENTION

In a first principal aspect, the present invention provides a homogeneous assay for the determination of aflatoxins in agricultural products. An aflatoxin extract is combined with a tracer and an antibody to provide a mixture. The antibody is specific for aflatoxin. The tracer comprises an aflatoxin oxime conjugated to a fluorophore selected from the group consisting of fluoresceinamine, 5-aminoacetyl-amidofluorescein, and 5-(5-aminopentyl)-thioureidyl fluorescein. The tracer is able to bind to the antibody to produce a detectable change in fluorescence polarization. The fluorescence polarization of the mixture is measured and compared to a standard curve.

In a second principle aspect, the present invention provides an assay kit for the determination of aflatoxins. The assay kit comprises an antibody and a tracer, each in an amount suitable for at least one assay, and suitable packaging. The antibody is specific for aflatoxin. The tracer comprises an aflatoxin oxime conjugated to a fluorophore selected from the group consisting of fluoresceinamine, 5-aminoacetyl-amidofluorescein, and 5-(5-aminopentyl)-thioureidyl fluorescein. The tracer is able to bind to the antibody to produce a detectable change in fluorescence polarization.

### BRIEF DESCRIPTION OF THE DRAWINGS

Figure 1 is a graph showing the change in fluorescence polarization over time for a range of aflatoxin concentrations, in accordance with an embodiment of the present invention.
Figure 2 is a graph showing the change in fluorescence polarization over time for a sample containing no aflatoxin and a range of methanol concentrations, using the data of Table 1, in accordance with an embodiment of the present invention.
Figure 3 is a standard curve for a fluorescence polarization assay for aflatoxins, using the data of Table 2, in accordance with an embodiment of the present invention.
Figure 4 is a graph comparing the aflatoxin concentration of samples as measured using HPLC with the aflatoxin concentration as calculated from the standard curve of Figure 3, in accordance with an embodiment of the present invention.
Figure 5 is graph comparing the aflatoxin concentration of spiked samples with the aflatoxin concentration calculated from measurements of fluorescence polarization, in accordance with an embodiment of the present invention.
Figure 6 is a standard curve for a fluorescence polarization assay for aflatoxins, used to obtain the data in Tables 6 and 7, in accordance with an embodiment of the present invention.

### DETAILED DESCRIPTION OF THE PREFERRED EMBODIMENTS

The preferred embodiments of the present invention provide a relatively simple homogeneous assay for the determination of aflatoxins in agricultural products that is based on measurements of fluorescence polarization. The technique of fluorescence polarization has been successfully utilized in various assay involving proteins, enzymes, drugs, DNA, hormones, peptides and antibodies.

The principle behind the fluorescence polarization technique is as follows. Fluorescent probes having low molecular weight have low polarization values due to their fast rotation, whereas fluorescent probes with higher molecular weight have higher polarization due to their slower rotation. Thus the polarization value of a fluorophore increases upon binding to a larger molecule. Further information about the fluorescence polarization technique is provided in U.S. Patent Nos. 5,427,960 and 5,976,820 and in Nasir, M. S. and Jolley, M. E., "Fluorescence Polarization: An analytical tool for Immunoassay and Drug Discovery," Combinatorial Chemistry & High Throughput Screening, 1999, 2, 177-190, which references are incorporated herein by reference.

In the present invention, aflatoxin extracted from a sample competes with a fluorescent tracer in the presence of a monoclonal antibody, thereby giving rise to a change in fluorescent polarization that is dependent upon the aflatoxin concentration.

The preferred embodiments of the present invention provide a homogeneous assay for aflatoxin that is sensitive, rapid, simple, and inexpensive. It can also be field-portable and yield quantitative results.

### 1. Materials and Methods

Two different aflatoxin monoclonal antibodies were used in these studies. An aflatoxin monoclonal antibody purchased from Sigma (catalog no. A-9555) was used in initial assay development work, but it was found to have sensitivity to methanol. In later work, a monoclonal antibody, available from Dr. Chris Maragos of the Agricultural Research Unit of the United States Department of Agriculture (Peoria, Illinois), was used because it was found to be stable in methanol. Use of this monoclonal antibody was reported in Chris M. Maragos and Vicki S. Thompson, "Fiber-optic Immunosensor for Mycotoxins," Natural Toxins 7:371-376 (1999). In addition, many other monoclonal antibodies for aflatoxins are known. *See, e.g.,* U.S. Patent No. 4,835,100.

Samples of corn that were naturally contaminated with aflatoxins, and samples of aflatoxin-free popcorn were purchased from Trilogy Analytical Laboratory, Inc. (Washington, Missouri). Trilogy also provided an Aflatoxin B₁/B₂/G₁/G₂ (7/1/3/1) mixture. Pure Aflatoxin B₁ was obtained from Sigma.

Fluorescence polarization measurements were done at room temperature using a single tube Sentry-FP fluorescence polarization instrument (Diachemix Corp.).

### 2. Preparation of Aflatoxin Tracer

In a 10 ml round bottomed flask fitted with a magnetic stirrer and a condenser, Aflatoxin B1 (5 mg, 0.016 mmol, Sigma) and O-carboxymethyl-hydroxylamine-hemihydrochloride (41 mg, 0.19 mmol, Sigma) were mixed with 1.2 ml absolute ethanol. To this solution, 230 µl of a 2 M NaOH solution (0.46 mmol) was added with stirring, and the solution refluxed for 3 hours. The resultant solution was stirred overnight at room temperature, concentrated on a rotary evaporator, and diluted to 1.5 ml with water. Drops of 1 N NaOH were added to adjust the pH to ~9, and the solution was washed with ethyl acetate (using two portions of about 3 ml each). The aqueous layer was acidified with 6 M HCl to a pH ~2, and the resultant mixture was stored at 0° C in a refrigerator. Some solid precipitated, which was separated and dried. TLC on silica using ethyl acetate:MeOH:NH₄OH (32:17:5) gave a major spot at Rf ~ 0.5 corresponding to the oxime product.

A 20 µl THF solution of (Aflatoxin B₁)-O-carboxymethyloxime, prepared as described above, was mixed with 20 µl of dicyclohexylcarbodiimide (DCC) in methylene chloride (10 mg/ml) and 200 µl of methylene chloride. After 2-3 minutes, a 20 µl THF solution of fluoresceinamine (isomer 2, 10 mg/ml) was added. The reaction was performed overnight at room temperature. As a control, the same reaction was also run without the aflatoxin oxime. TLC of the products (using CHCl₃:MeOH:CH₃CO₂H, 40:10:3) showed many spots. One spot at Rf~0.7 was found to be absent in the TLC run of the control. This tracer was collected, dissolved in MeOH, and then diluted in buffer so as to give an intensity of~400,000 relative fluorescence units when 10 µl of this tracer solution was added to 1 ml of buffer.

It was observed that this tracer gave a stable polarization of ~40 mP. After adding 10 µl of 1/50 diluted antibody (Sigma, A-9555) to give a final dilution of 1/5,000, the polarization slowly increased to ~230 mP in a period of five minutes.

In order to confirm the reactivity, 1 ml buffer was taken and mixed with 10 µl of antibody and 10 µl of (0.8 mg/ml) Aflatoxin B₁. The mixture was kept at room temperature for five minutes and then blanked in the FP instrument. After 10 µl of tracer was added, the fluorescence polarization decreased from ~230 mP to ~41 mP.

A different Aflatoxin B₁ tracer was prepared using a similar reaction but with fluoresceinamine (isomer 1) as the fluorophore. The resulting tracer showed less sensitivity than when fluoresceinamine (isomer 2) was used as the fluorophore. Specifically, the starting polarization (~32mP) changed to only ~140 mP when the antibody was added.

Aflatoxin B₁ tracers were also prepared using other amine derivatives of fluorescein as the fluorophore. The results are summarized as follows. When 5-aminoacetyl-amidofluorescein (5AAF) was used, the resulting tracer had a fluorescence polarization of ~30 mP, which increased to ~65 mP when antibody was added. When 5-(5-aminopentyl)-thioureidyl fluorescein (5,5APTF) was tried as the fluorophore, the tracer had a fluorescence polarization of~35 mP, which increased to ~102 mP upon antibody addition. Fluorescein thiosemicarbazide (FTSC), 4-aminomethyl fluorescein, and 5-aminomethyl fluorescein were also tried, but these fluorophores did not result in any active product, i.e., they showed no significant change in fluorescence polarization upon binding to antibody. Therefore, it was concluded from these studies that isomer 2 of fluoresceinamine gave the best results for this antibody.

### 3. Assay Development

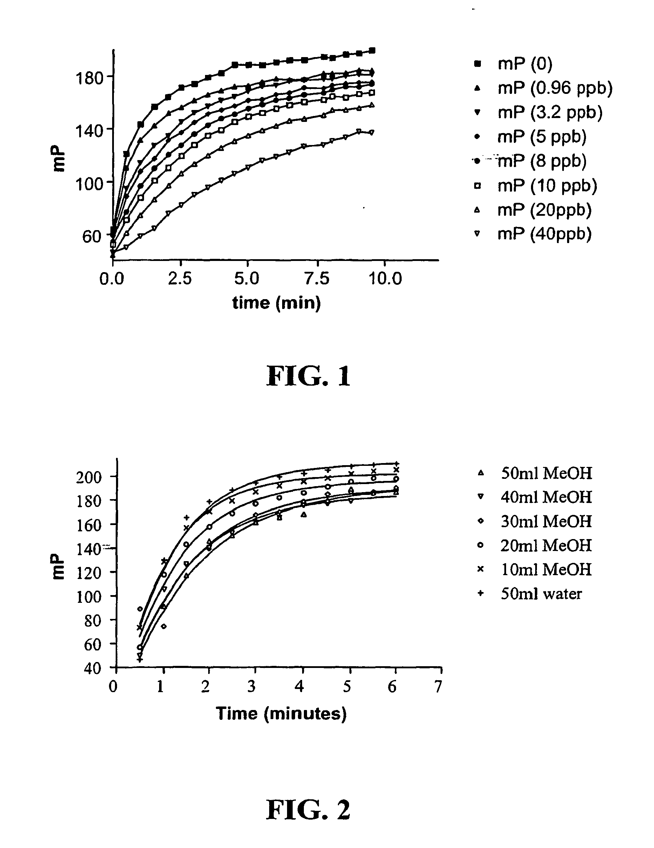

Aflatoxin antibody was purchased from Sigma (A-9555) and used for initial assay development after diluting it (1/150,000) in PBSA-BGG (pH ~7.5), which is a phosphate buffer solution containing 1 gram per liter sodium azide and 9 grams per liter sodium chloride, with bovine gamma globulin (BGG) present at a concentration of 100µg/ml. 1 ml of the antibody solution was mixed with 50 µl of a solution having a known concentration of free Aflatoxin B₁ in methanol/water (70/30). Aflatoxin B₁ concentrations varying from 0 to 40 ppb were used. After taking a blank measurement, 10 µl of diluted tracer, prepared as described above using fluoresceinamine (isomer 2) as the fluorophore, was added, and the change in fluorescence polarization was monitored for 10 minutes. The results are shown in Figure 1.

Since aflatoxins are typically extracted from agricultural samples using a mixture of an organic solvent and water, the effect of methanol on the Aflatoxin antibody from Sigma (A-9555) was studied. Specifically, 1 ml of diluted antibody solution was mixed with a known concentration of methanol. After measuring a blank, tracer was added (the Aflatoxin B₁ oxime-isomer 2 fluoresceinamine tracer described above), and the fluorescence polarization was measured over time. The results are summarized in Table 1 below and in Figure 2.

**TABLE 1**

| **Time (Minutes)** | 50ml MeOH | 40ml MeOH | 30ml MeOH | 20ml MeOH | 10ml MeOH | 50ml water |
|---|---|---|---|---|---|---|
| 0.5 | 32.0 | 49.70 | 88.80 | 56.70 | 73.20 | 46.30 |
| 1.0 | 91.0 | 105.40 | 74.40 | 117.60 | 128.40 | 129.80 |
| 1.5 | 117.0 | 126.20 | 126.30 | 143.00 | 156.70 | 165.40 |
| 2.0 | 145.9 | 140:50 | 139.30 | 157.70 | 170.40 | 178.60 |
| 2.5 | 150.4 | 152.50 | 154.30 | -168.90 | -179.20 | 188.30 |
| 3.0 | 161.3 | 161.50 | 167.50 | 176.70 | 186.80 | 194.30 |
| 3.5 | 165.5 | 165.70 | 170.30 | 181.90 | 192.10 | 199.60 |
| 4.0 | 168.1 | 175.40 | 178.60 | 186.10 | 195.50 | 202.30 |
| 4.5 | 179.6 | 177.10 | 185.10 | 191.30 | 198.70 | 205.40 |
| 5.0 | 189.3 | 179.10 | 186.80 | 195.50 | 202.10 | 208.30 |
| 5.5 | 186.1 | 185.80 | 187.10 | 198.40 | 204.50 | 209.80 |
| 6.0 | 186.6 | 187.00 | 190.40 | 198.00 | 205.60 | 210.60 |

These results show that the fluorescence polarization decreases as the methanol concentration increases, and, for a given methanol concentration, the fluorescence polarization increases over time. Thus, although the tracer itself is stable in methanol and related organic solvents for an extended period of time, these results suggest that this antibody is sensitive to methanol. As a result, while this antibody has the sensitivity for use in a fluorescence polarization-based assay for aflatoxins, it is preferable to use an antibody that is more stable in methanol in order to yield more reliable results. It was found that the monoclonal antibody available from Dr. Chris Maragos of the Agricultural Research Unit of the United States Department of Agriculture (Peoria, Illinois) had the desired stability in methanol.

### 4. FP Assay for Aflatoxins in Naturally Contaminated Corn Samples

Corn samples that were naturally contaminated with aflatoxins were purchased from Trilogy Analytical Laboratory, Inc. (Washington, Missouri). Aflatoxin was extracted from each 20 g sample of crushed grain using 100 ml mixture of MeOH/water (70/30) in duplicate by shaking each sample from time to time for about 30 minutes. Extracts were filtered through a fine filter paper and stored in sealed bottles at room temperature for analysis.

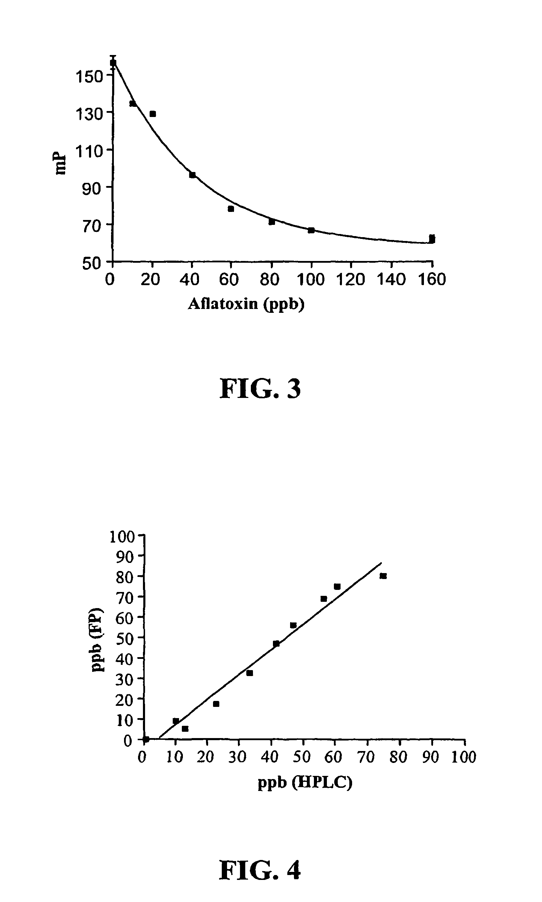

Standards were prepared in MeOH/water (70/30) by diluting a concentrate of Aflatoxin B₁/B₂/G₁/G₂ (7/1/3/1) provided by Trilogy into various concentrations. 40 µl of each sample or standard was mixed into 1 ml antibody solution (1/150,000 in PBSA-BGG buffer) in a test tube. The antibody used was the methanol resistant antibody provided by Dr. Chris Maragos. After blanking each sample, 10 µl of tracer was added into each tube, the samples were incubated for 15 minutes at room temperature, and then the fluorescence polarization was measured for each tube. The tracer that was used was the Aflatoxin B₁ oxime-isomer 2 fluoresceinamine tracer described above. A standard curve was plotted using duplicate values. The fluorescence polarization values for the standards are shown below in Table 2 and in Figure 3.

**TABLE 2**

| **Aflatoxin Concentration (ppb)** | **mP (first run)** | **mP (second run)** |
|---|---|---|
| 0.0 | 153.0 | 160.0 |
| 10.0 | 134.0 | 135.0 |
| 20.0 | 128.0 | 130.0 |
| 40.0 | 97.0 | 96.0 |
| 60.0 | 78.0 | 79.0 |
| 80.0 | 71.0 | 72.0 |
| 100.0 | 67.0 | 67.0 |
| 160.0 | 64.0 | 60.0 |

The concentration of aflatoxin in each corn sample was then calculated from the standard curve. The results are summarized in Table 3 below.

**TABLE 3**

| **Sample** | **mP (first run)** | **mP (second run)** | **Calculated aflatoxin concentration (ppb)** |
|---|---|---|---|
| 1 | 163.0 | 159.0 | 0 |
| 2 | 143.5 | 135.0 | 8.99 |
| 3 | 80.0 | 75.5 | 69 |
| 4 | 92.0 | 90.5 | 47 |
| 5 | 98.0 | 112.5 | 32.61 |
| 9 | 73.5 | 76.5 | 75.16 |
| 7 | 75.5 | 71.0 | 79.94 |
| 8 | 83.0 | 87.0 | 56 |
| 10 | 125.0 | 124.5 | 17.35 |
| 11 | 146.5 | 146.0 | 5.4 |

These samples were also analyzed using both the fluorescence polarization protocol described above and by standard HPLC techniques, and the aflatoxin concentrations determined using these two techniques were compared. The results are summarized below in Table 4 and in Figure 4, except for the results from one sample that had a very high level of aflatoxin contamination. These results show a good correlation between HPLC and FP (*r*² = 0.97).

**TABLE 4**

| **Aflatoxin concentration from HPLC (ppb)** | **Aflatoxin concentration from FP (ppb)** |
|---|---|
| 0.50 | 0.0 |
| 10.20 | 9.0 |
| 56.40 | 69.0 |
| 41.50 | 47.0 |
| 33.30 | 32.6 |
| 47.10 | 56.0 |
| 60.80 | 75.0 |
| 75.00 | 80.0 |
| 13.01 | 5.4 |

### 5. Analysis Aflatoxin in Popcorn Samples

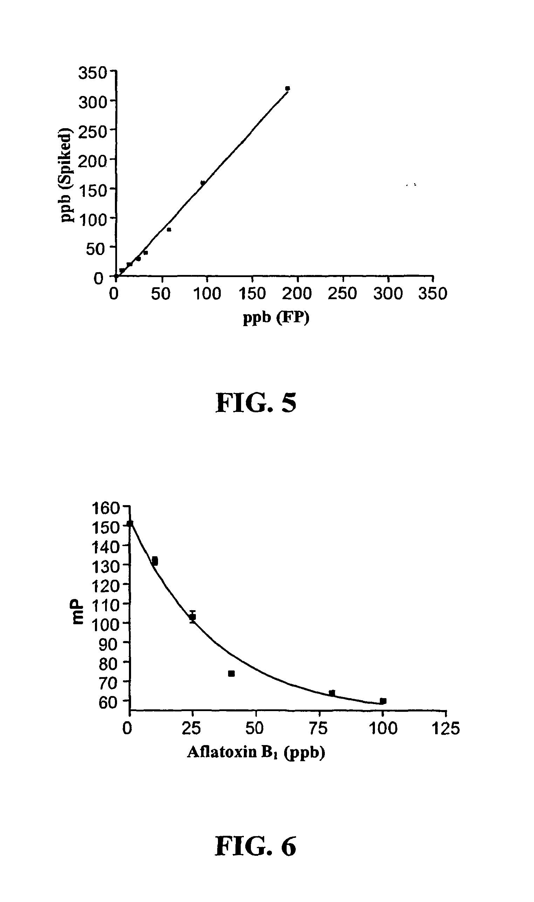

20 g of crushed samples of aflatoxin-free popcorn were spiked with an Aflatoxin B₁/B₂/G₁/G₂ mixture (7/1/3/1) to a known concentration. The fluorescence polarization analysis was performed in duplicate on each extract as reported above. The aflatoxin concentration of each sample was calculated from the average measured fluorescence polarization, using a calibration curve, and compared with the known spiked concentration. The results are summarized in Table 5 below and in Figure 5. In this study, the sample spiked to 320 ppb was diluted 1/10 for the fluorescence polarization measurement.

**TABLE 5**

| **Spiked Aflatoxin Concentration (ppb)** | **mP** | **Calculated Aflatoxin Concentration (ppb)** |
|---|---|---|
| 0 | 178 | 0.47 |
| 10 | 169 | 5.79 |
| 10 | 168 | 6.43 |
| 20 | 157 | 14.60 |
| 20 | 159 | 13.16 |
| 30 | 148 | 23.30 |
| 40 | 142 | 31.12 |
| 80 | 129 | 57.72 |
| 160 | 122 | 94.74 |
| 320 | 152 | 189 |

These results show a good correlation between theoretical values and the results obtained using the fluorescence polarization assay of the present invention (r² = 0.996). However, the fluorescence polarization results consistently underestimated the actual aflatoxin concentration. One explanation is that naturally contaminated samples had mainly B₁ and some B₂, but no G₁ and G₂, whereas the popcorn samples were spiked with a mixture of B₁/B₂/G₁/G₂ in a 7/1/3/1 ratio.

To test this explanation, the cross-reactivity of these aflatoxins was investigated. Aflatoxin B₁, B₂, G₁ and G₂ were purchased from Sigma and individually diluted in a mixture of methanol/water (70/30) to give a range of concentrations, namely, 10, 25, 40, 80, and 100 ppb. The standard curve shown in Figure 6 was obtained by performing the fluorescence polarization assay described herein on the Aflatoxin B₁ solutions. Table 6 and Table 7 show the results for Aflatoxin G₁ and G₂, respectively, calculating the aflatoxin concentrations from the calibration curve of Figure 6.

**TABLE 6**

| **Aflatoxin G₁ Concentration (ppb)** | **mP** | **Calculated Aflatoxin Concentration (ppb)** |
|---|---|---|
| 10 | 143 | 3.75 |
| 25 | 130 | 9.06 |
| 40 | 115 | 16.44 |
| 80 | 101 | 25.16 |
| 100 | 99 | 26.61 |

**TABLE 7**

| **Aflatoxin G₂ Concentration (ppb)** | **mP** | **Calculated Aflatoxin Concentration (ppb)** |
|---|---|---|
| 10 | 144 | 3.37 |
| 25 | 133 | 7.76 |
| 40 | 113 | 17.56 |
| 80 | 101 | 25.16 |
| 100 | 98 | 27.35 |

These results show that the concentrations of Aflatoxin G₁ and G₂ are underestimated when they are calculated from a calibration curve obtained from Aflatoxin B₁ alone. More particularly, Aflatoxin G₁ and G₂ both cross-react with Aflatoxin B₁ only to the extent of 30-40%. This may explain the underestimation of aflatoxin concentration observed in the spiked popcorn samples.

### 6. Assay Kit

The materials used to perform the assay of the present invention are preferably made available in kit form. The kit preferably includes a quantity of extraction solution for extracting aflatoxin from samples of grain or other products, tracer and antibody in an amount suitable for at least one assay, along with suitable packaging and instructions for use. The tracer and antibody may be provided in solution, as a liquid dispersion, or as a substantially dry powder (e.g., in lyophilized form).

The suitable packaging can be any solid matrix or material, such as glass, plastic, paper, foil, and the like, capable of separately holding within fixed limits the buffer, tracer, and antibody. For example, the extraction solvent, tracer, and monoclonal antibody may be provided as solutions in separate labeled bottles or vials made of glass or plastic.

The antibody is specific for aflatoxins and is preferably a monoclonal antibody. Most preferably, the monoclonal antibody is stable in the extraction solvent.

The tracer comprises a fluorophore conjugated to an aflatoxin oxime, preferably (Aflatoxin B₁)-O-carboxymethyloxime. Suitable fluorophores are fluoresceinamine (isomer 1), fluoresceinamine (isomer 2), 5-aminoacetyl-amidofluorescein (5AAF), and 5-(5-aminopentyl)-thioureidyl fluorescein (5,5APTF). Preferably the fluorophore is fluoresceinamine. Most preferably, the fluorophore is fluoresceinamine (isomer 2).

The extraction solvent is preferably a mixture of an organic solvent, such as methanol or acetonitrile, in water. Most preferably, the extraction solvent is a methanol/water (70/30) mixture.

### 7. References

(1) Langone, J. J.; Vunakis, H. V. "Aflatoxin B1: Specific antibodies and their use in radioimmunoassays." J. Natl. Cancer Inst. 1976, 56, 591-595.
(2) Sizaret, P.; Malaveille, C.; Montesano, R.; Frayssinet, C. "Detection of Aflatoxins and related metabolites by radioimmunoassay." J. Natl. Cancer Inst. 1982, 69, 1375-1381.
(3) Whitaker, T.; Horwitz, W.; Albert, R.; Nesheim, S. "Variability associated with analytical methods used to measure aflatoxin in agricultural commodities." J. AOAC Int. 1996, 79, 476-485.
(4) Wilson, T. J.; Romer, T. R. "Mycotoxins: Use of the mycosep multifunctional cleanup column for liquid chromatographic determination of Aflatoxins in agricultural products." J. Assoc. Off. Anal. Chem. 1991, 74, 951-956.
(5) Newbeme, P. M.; Butler, W. H. Cancer Res. 1969, 29, 236-250.
(6) Trucksess, M. W.; Stack, M. E.; Nesheim, S.; Page, S. W.; Albert, R. H.; Hansen, T. J.; Donahue, K. F. "Immunoaffinity column coupled with solution fluorometry or liquid chromatography postcolumn derivatization for determination of aflatoxins in corn, peanuts and peanut butter: Collaborative study." J. Assoc. Off. Anal. Chem. 1991, 74, 81-88.
(7) Wild, C. P.; Pionneau, F. A.; Montesano, R.; Mutiro, C. F.; Chetsanga, C. J. "Aflatoxin detection in human breast milk by immunoassay." Int. J. Cancer. 1987, 40, 328-333.
(8) Trucksess, M. W.; Stack, M. E.; Nesheim, S.; Albert, R. H.; Romer, T. R. "Multifunctional column coupled with liquid chromatography for determination of aflatoxins B1, B2, G1 and G2 in corn, almonds, brazil nuts, peanuts and pistachio nuts: Collaborative study." J. AOAC. Int. 1994, 77, 1512-1521.
(9) Campbell, T. C.; Stoloff, L. "Implication of mycotoxins for human health." J. Agr. Food Chem. 1974, 22, 1006-1014.
(10) Seitz, L. M. "Comparison of methods of aflatoxin analysis by high-pressure liquid chromatography." J. Chromatogr. 1975, 104, 81-91.
(11) Asao, T.; Buchi, G.; Abdel-kader, M. M.; Chang, S. B.; Wick, E. L.; Wogan, G. N. "The structure of Aflatoxins B and G1." J. Am. Chem. Soc. 1965, 87, 882-886.

## Claims

1. A homogeneous assay for the determination of aflatoxins in agricultural products, said homogeneous assay comprising the steps of:
combining an aflatoxin extract with a tracer and an antibody to provide a mixture, said antibody being specific for aflatoxin, said tracer comprising an aflatoxin oxime conjugated to a fluorophore selected from the group consisting of fluoresceinamine, 5-aminoacetyl-amidofluorescein, and 5-(5-aminopentyl)-thioureidyl fluorescein, said tracer being able to bind to said antibody to produce a detectable change in fluorescence polarization;
measuring the fluorescence polarization of said mixture to obtain a measured fluorescence polarization; and
comparing said measured fluorescence polarization with a **characterized** fluorescence polarization value, said **characterized** fluorescence polarization value corresponding to a known aflatoxin concentration.

2. The assay of claim 1, wherein said fluorophore is an isomer of fluoresceinamine.

3. The assay of claim 2, wherein said fluorophore is isomer 2 of fluoresceinamine.

4. The assay of claim 1, wherein said aflatoxin oxime is (Aflatoxin B₁)-O-carboxymethyloxime.

5. The assay of claim 1, further comprising the steps of:
providing a plurality of aflatoxin standard solutions, each of said aflatoxin standard solutions having a different known concentration of aflatoxin;
adding said tracer and said antibody to each one of said plurality of aflatoxin standard solutions, so as to provide a plurality of standard mixtures; and
measuring the fluorescence polarization of each one of said plurality of said standard mixtures to provide a plurality of standard fluorescence polarization values corresponding to known aflatoxin concentrations.

6. The assay of claim 5, wherein said **characterized** fluorescence polarization value is one of said standard fluorescence polarization values.

7. An assay kit for the determination of aflatoxins in agricultural products, said assay kit comprising:
an antibody and a tracer, each in an amount suitable for at least one assay, and suitable packaging, said antibody being specific for aflatoxin, said tracer comprising an aflatoxin oxime conjugated to a fluorophore selected from the group consisting of fluoresceinamine, 5-aminoacetyl-amidofluorescein, and 5-(5-aminopentyl)-thioureidyl fluorescein.

8. The assay kit of claim 7, wherein said fluorophore is fluoresceinamine.

9. The assay kit of claim 8, wherein said fluorophore is isomer 2 of fluoresceinamine.

10. The assay kit of claim 7, wherein said aflatoxin oxime is (Aflatoxin B₁)-O-carboxymethyloxime.

11. An aflatoxin oxime conjugated to a fluorophore selected from the group consisting of fluoresceinamine, 5-aminoacetyl-amidofluorescein, and 5-(5-aminopentyl)-thioureidyl fluorescein for carrying out the assay according to any one of claims 1 to 6.

12. The aflatoxin oxime of claim 11, wherein said fluorophore is an isomer of fluoresceinamine.

13. The aflatoxin oxime of claim 12, wherein said fluorophore is an isomer 2 of fluoresceinamine.

14. The aflatoxin oxime of claim 11, wherein said aflatoxin oxime is (Aflatoxin B₁)-O-carboxymethyloxime.

15. Use of the aflatoxin oxime of any one of claims 11 to 14 for the preparation of an assay kit for the determination of aflatoxins by fluorescence polarization.

16. The use of claim 15, wherein the preparation additionally includes an antibody specific for aflatoxin.

## Patentansprüche

1. Homogener Assay zur Bestimmung von Aflatoxinen in landwirtschaftlichen Produkten, wobei der homogene Assay die folgenden Schritte umfasst:
Vereinigen eines Aflatoxin-Extrakts mit einem Tracer und einem Antikörper, um eine Mischung bereitzustellen, wobei der Antikörper für Aflatoxin spezifisch ist, wobei der Tracer ein Aflatoxin-Oxim umfasst, das mit einem Fluorophor konjugiert ist, der aus der Gruppe bestehend aus Fluoresceinamin, 5-Aminoacetylamidofluorescein und 5-(5-Aminopentyl)-thioureidylfluorescein ausgewählt ist, wobei der Tracer in der Lage ist, den Antikörper zu binden, um eine nachweisbare Änderung in der Fluoreszenzpolarisation zu erzeugen;
Messen der Fluoreszenzpolarisation der Mischung, um eine gemessene Fluoreszenzpolarisation zu erhalten; und
Vergleichen der gemessenen Fluoreszenzpolarisation mit einem charakteristischen Fluoreszenzpolarisationswert, wobei der charakteristische Fluoreszenzpolarisationswert einer bekannten Aflatoxin-Konzentration entspricht.

2. Assay nach Anspruch 1, wobei der Fluorophor ein Isomer des Fluoresceinamins ist.

3. Assay nach Anspruch 2, wobei der Fluorophor das Isomer 2 des Fluoresceinamins ist.

4. Assay nach Anspruch 1, wobei das Aflatoxin-Oxim (Aflatoxin-B₁)-O-carboxymethyloxim ist.

5. Assay nach Anspruch 1, zusätzlich umfassend die Schritte:
Bereitstellen einer Mehrzahl an Aflatoxin-Standardlösungen, wobei jede der Aflatoxin-Standardlösungen eine unterschiedliche, bekannte Aflatoxin-Konzentration aufweist;
Hinzufügen des Tracers und des Antikörpers zu jeder der Vielzahl von Aflatoxin-Standardlösungen, um eine Vielzahl an Standardmischungen bereitzustellen; und
Messen der Fluoreszenzpolarisation jeder der Vielzahl der Standardmischungen, um eine Vielzahl an Standard-Fluoreszenzpolarisationswerten bereitzustellen, die bekannten Aflatoxin-Konzentrationen entsprechen.

6. Assay nach Anspruch 5, wobei der charakteristische Fluoreszenzpolarisationswert einer der Standardfluoreszenz-Polarisationswerte ist.

7. Assay-Kit zur Bestimmung von Aflatoxinen in landwirtschaftlichen Produkten, wobei das Assay-Kit umfasst:
einen Antikörper und einen Tracer, jeweils in einer für wenigstens einen Assay geeigneten Menge, und eine geeignete Verpackung, wobei der Antikörper für Aflatoxin spezifisch ist, wobei der Tracer ein Aflatoxin-Oxim umfasst, das mit einem Fluorophor konjugiert ist, der aus der Gruppe bestehend aus Fluoresceinamin, 5-Aminoacetyl-amidofluorescein und 5-(5-Aminopentyl)-thioureidylfluorescein ausgewählt ist, wobei der Tracer in der Lage ist, den Antikörper zu binden, um eine nachweisbare Änderung in der Fluoreszenzpolarisation zu erzeugen.

8. Assay-Kit nach Anspruch 7, wobei der Fluorophor Fluoresceinamin ist.

9. Assay-Kit nach Anspruch 8, wobei der Fluorophor das Isomer 2 des Fluoresceinamins ist.

10. Assay-Kit nach Anspruch 7, wobei das Aflatoxin-Oxim (Aflatoxin-B₁)-O-carboxymethyloxim ist.

11. Aflatoxin-Oxim, das mit einem Fluorophor konjugiert ist, der aus der Gruppe bestehend aus Fluoresceinamin, 5-Aminoacetyl-amidofluorescein und 5-(5-Aminopentyl)-thioureidylfluorescein ausgewählt ist, um den Assay gemäß einem beliebigen der Patentansprüche 1 bis 6 durchzuführen.

12. Aflatoxin-Oxim nach Anspruch 11, wobei der Fluorophor ein Isomer des Fluoresceinamins ist.

13. Aflatoxin-Oxim nach Anspruch 12, wobei der Fluorophor das Isomer 2 des Fluoresceinamins ist.

14. Aflatoxin-Oxim nach Anspruch 11, wobei das Aflatoxin-Oxim (Aflatoxin-B₁)-O-carboxymethyloxim ist.

15. Verwendung des Aflatoxin-Oxims nach einem beliebigen der Ansprüche 11 bis 14 zur Präparation eines Assay-Kits zur Bestimmung von Aflatoxinen durch Fluoreszenzpolarisation.

16. Verwendung nach Anspruch 15, wobei die Präparation zusätzlich einen für Aflatoxin spezifischen Antikörper umfasst.

## Revendications

1. Dosage homogène pour la détermination d'aflatoxines dans des produits agricoles, ledit dosage homogène comprenant les étapes de:
combinaison d'un extrait d'aflatoxine avec un traceur et un anticorps pour procurer un mélange, ledit anticorps étant spécifique pour l'aflatoxine, ledit traceur comprenant une aflatoxine oxime conjuguée à un fluorophore choisi dans le groupe consistant en fluorescéinamine, 5-aminoacétyl-amidofluorescéine, et 5-(5-aminopentyl)-thiouréïdyl fluorescéine, ledit traceur étant capable de se lier au dit anticorps pour produire une modification détectable dans la polarisation de fluorescence;
mesure de la polarisation de fluorescence dudit mélange pour obtenir une polarisation de fluorescence mesurée; et
comparaison de ladite polarisation de fluorescence mesurée avec une valeur de polarisation de fluorescence **caractérisée**, ladite valeur de polarisation de fluorescence **caractérisée** correspondant à une concentration d'aflatoxine connue.

2. Dosage selon la revendication 1, dans lequel ledit fluorophore est un isomère de fluorescéinamine.

3. Dosage selon la revendication 2, dans lequel ledit fluorophore est l'isomère 2 de la fluorescéinamine.

4. Dosage selon la revendication 1, dans lequel ladite aflatoxine oxime est l'(aflatoxine B₁)-O-carboxyméthyloxime.

5. Dosage selon la revendication 1, comprenant en outre les étapes de:
fourniture d'une pluralité de solutions standard d'aflatoxine, chacune desdites solutions standard d'aflatoxine ayant une concentration connue différente d'aflatoxine;
addition dudit traceur et dudit anticorps à chacune des solutions standard d'aflatoxine de ladite pluralité, de manière à procurer une pluralité de mélanges standard; et
mesure de la polarisation de fluorescence de chacun des dits mélanges standard de ladite pluralité de mélanges standard pour procurer une pluralité de valeurs de polarisation de fluorescence standard correspondant à des concentrations d'aflatoxine connues.

6. Dosage selon la revendication 5, dans lequel ladite valeur de polarisation de fluorescence **caractérisée** est l'une desdites valeurs de polarisation de fluorescence standard.

7. Kit de dosage pour la détermination d'aflatoxines dans des produits agricoles, ledit kit de dosage comprenant:
un anticorps et un traceur, chacun en une quantité appropriée pour au moins un dosage, et un emballage approprié, ledit anticorps étant spécifique pour l'aflatoxine, ledit traceur comprenant une aflatoxine oxime conjuguée à un fluorophore choisi dans le groupe consistant en fluorescéinamine, 5-aminoacétyl-amidofluorescéine, et 5-(5-amino-pentyl)-thiouréïdyl fluorescéine, ledit traceur étant capable de se lier au dit anticorps pour produire une modification détectable dans la polarisation de fluorescence.

8. Kit de dosage selon la revendication 7, dans lequel ledit fluorophore est la fluorescéinamine.

9. Kit de dosage selon la revendication 8, dans lequel ledit fluorophore est l'isomère 2 de la fluorescéinamine.

10. Kit de dosage selon la revendication 7, dans lequel ladite aflatoxine oxime est l'(aflatoxine B₁)-O-carboxyméthyloxime.

11. Aflatoxine oxime conjuguée à un fluorophore choisi dans le groupe consistant en fluorescéinamine, 5-aminoacétyl-amidofluorescéine, et 5-(5-aminopentyl)-thiouréidyl fluorescéine, pour la réalisation du dosage selon l'une quelconque des revendications 1 à 6.

12. Aflatoxine oxime selon la revendication 11, dans laquelle ledit fluorophore est un isomère de fluorescéinamine.

13. Aflatoxine oxime selon la revendication 12, dans laquelle ledit fluorophore est l'isomère 2 de la fluorescéinamine.

14. Aflatoxine oxime selon la revendication 11, dans laquelle ladite aflatoxine oxime est 1'(aflatoxine B₁)-O-carboxyméthyloxime.

15. Utilisation de l'aflatoxine oxime selon l'une quelconque des revendications 11 à 14 pour la préparation d'un kit de dosage pour la détermination d'aflatoxines par polarisation de fluorescence.

16. Utilisation selon la revendication 15, dans laquelle la préparation comporte en outre un anticorps spécifique pour l'aflatoxine.
